(19) **Europäisches Patentamt / European Patent Office / Office européen des brevets**

(11) **EP 4 712 088 A1**

(12) **EUROPEAN PATENT APPLICATION**

(43) Date of publication:
**18.03.2026 Bulletin 2026/12**

(21) Application number: **24306487.0**

(22) Date of filing: **11.09.2024**

(51) International Patent Classification (IPC):
**G16C 20/10** (2019.01)    **G16C 20/70** (2019.01)
**G16C 60/00** (2019.01)

(52) Cooperative Patent Classification (CPC):
**G16C 20/10; G06N 3/08; G06N 5/01; G06N 20/00;
G06N 20/20; E04C 2/00; G06N 3/00; G16C 20/70;
G16C 60/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **SAINT-GOBAIN PLACO**
**92400 Courbevoie (FR)**

(72) Inventors:
• **CHARRAUD, Jean-Baptiste**
**93300 Aubervilliers (FR)**

• **LOUREAU, Jeanne**
**92400 Courbevoie (FR)**
• **ROUYRE-READ, Thomas**
**92400 Courbevoie (FR)**
• **CASTILLA GARCIA, Carlos**
**28330 San Martin de la Vega (ES)**
• **ORTEGO MATE, Jesus**
**28330 San Martin de la Vega (ES)**
• **LE BIHAN, Etienne**
**92400 Courbevoie (FR)**

(74) Representative: **Murgitroyd & Company**
**165-169 Scotland Street**
**Glasgow G5 8PL (GB)**

(54) **COMPUTER-IMPLEMENTED METHODS AND SYSTEM FOR CALCINATION PROCESSES**

(57)    A computer-implemented method is provided which enables a user to utilise historical data to optimise the configuration to a drying environment.

$$CT = \sum_{i=1}^{N} \frac{dS_i}{dt} + S_{flow}$$

Fig. 1

## Description

### FIELD

The invention relates to a method and system. Particularly, but not exclusively, the invention relates to a computer-implemented method and system. Particularly, but not exclusively, the invention relates to a computer-implemented method of estimating calcination throughput from a calcination environment.

### BACKGROUND

Plasterboard is a widely used building material. When used in construction, plasterboard assist builders and designers in the control of condensation, fire protection and insulation (both thermal and acoustic). Plasterboard is usually used to make internal walls and ceilings.

Plasterboard comprises a core between two facers. The facers are usually either heavy duty construction paper or glass fibre mats where the glass fibre mats may be fully or partially embedded in the gypsum. The core comprises mostly calcium sulfate dihydrate, also known as gypsum. The plasterboard is formed depositing a wet slurry onto a first facer, before adding the second facer on top. The slurry comprises water, calcium sulfate hemihydrate, also known as stucco, and other additives such as foam and setting accelerators.

Estimating the quantity of stucco produced from raw gypsum is key in controlling a production environment for plasterboard as it enables the prediction of the amount of plasterboard which will be output from a production environment. The amount of stucco can also indicate the performance or efficiency of the calcination environment.

Aspects and embodiments of the invention were conceived with the foregoing in mind.

### SUMMARY

Aspects of the invention relate to calcination environments, where raw gypsum is taken as input to the calcination environment and placed in a calciner or a furnace and turned into stucco, which is then used in the production process used to produce gypsum products such as, for example, plasterboard and setting material (which may also be described as jointing material or smoothing plaster). The calcination throughput, i.e. the amount of stucco being produced by the calcination environment, is estimated by aspects of the invention and also used to optimise a calcination environment.

Viewed from a first aspect of the invention, there is provided a computer-implemented method of estimating calcination throughput from a calcination environment. The method may be implemented by a processing resource. The processing resource may be implemented by software, hardware or a mixture of both. The processing resource may be implemented by a cloud-based resource. The processing resource may be configured to receive data from and transmit data to the calcination environment. This may be by any suitable telecommunications protocol.

The method may comprise initialising a trained model. The initialisation of the trained model may comprise initialising and allocating the software and/or hardware resources needed to implement the trained model.

The trained model may be trained to predict a calcination throughput from a input associated with a calcination environment. The model may be trained using historical calcination data. The historical calcination data may comprise measurements related to one or more variables which can be measured from the calcination environment.

The method may further comprise receiving at least one calcination parameter associated with the calcination environment. The calcination parameter may be received in real-time and may be received as part of a request for an estimate of calcination throughput. The request may be received from a calcination environment without user input (i.e. automatically) or it may be received responsive to user input which may be received from an operative. The user input may be input via a user interface in communication with the processing resource.

The method may comprise applying the trained model to the at least one calcination parameter. The calcination parameter may be any parameter which is taken from the calcination environment (e.g. gas valve percentage). The calcination parameter may be received in real time (e.g. on a second-by-second basis). The calcination parameter may be received in a real time data feed from the calcination environment.

The method may further comprise obtaining, from the trained model, a calcination throughput estimate for the calcination environment by using the at least one calcination parameter as an input to the trained model.

A method in accordance with the first aspect enables a calcination throughput estimate to be provided in real-time using calcination parameters taken from the calcination environment. A trained model is used which provides a calcination throughput estimate using a calcination parameter received from the calcination environment.

Optionally, the trained model comprises a regression based estimator, which may be a gradient boosting tree regressor. The effect of this is that the calcination throughput is used as the output in the training of the model and this enables there to be provided which is simple to implement and which enables the calcination throughput to be predicted based on calcination parameters.

The training of the trained model may comprise supervised learning where production parameters associated with calcination environments and calcination throughputs are used as respective labelled input-output pairs.

The at least one calcination parameter may comprise at least one of mill inlet pressure, calcination temperature, gas consumption, output aperture position, mill outlet pressure, combustion air temperature, rock feeding rate, recirculation air flow, main fan power, stucco cooler outlet temperature and mill pressure.

The at least one calcination parameter may be obtained autonomously from the calcination environment.

The at least one calcination parameter may be obtained from the calcination environment responsive to human input.

The calcination throughput estimate may be provided to a display. This may be via a user interface on the display. The display may be mounted to a calcination environment or it may be located remotely relative to the calcination environment.

Viewed from a second aspect of the invention, there may be provided a computer-implemented method of generating a configuration for a calcination environment. A configuration may be understood to mean a collection of parameters which can be used to set the physical environment which is to be used for calcination.

The method may be implemented by a processing resource. The processing resource may be implemented by software, hardware or a mixture of both. The processing resource may be implemented by a cloud-based resource. The processing resource may be configured to receive data from and transmit data to the calcination environment. This may be by any suitable telecommunications protocol.

The method may comprise obtaining historical calcination data, wherein the historical calcination data comprises measurements related to a plurality of directly adjustable and indirectly adjustable variables. The directly adjustable variable may be variables which can be controlled directly from a user interface by an operative. The indirectly adjustable variables may be variables which are directly adjusted based on the directly adjustable variables.

The historical calcination data may be separated into a training and testing data set. The historical calcination data may be taken during a specific production period.

The method may converge on a set of optimised calcination parameters by:

i) identifying relationships between at least one directly adjustable variable and an indirectly adjustable variable based on the historical calcination data;

ii) identifying a correlation between at least one directly adjustable variable and a quality parameter

iii) determining a variation in quality in the plasterboard based on at least one adjustable variable using the quality parameter;

iv) determining a calcination threshold prediction based on the identified indirectly adjustable variable and directly adjustable variable;

v) establishing a quality score based on the calcination threshold and the variation in quality;

vi) repeating steps iii) to v) until successive quality scores differ by less than a threshold amount;

vii) outputting the calcination parameters corresponding to the step where the quality score differs from the previous quality score by less than the threshold amount.

The identification of relationships between directly and indirectly adjustable variables may be implemented using any suitable regression technique e.g. Ridge Regression. This enables the establishment of linear relationships between directly and indirectly adjustable variables.

The identifying of a correlation between at least one directly adjustable variable and a quality parameter may be implemented by establishing a Pearson coefficient between the respective variables which describes the linear relationship between the respective variables. The quality parameters may be identified by an operative of the calcination environment.

Determining a variation in quality in the plasterboard may based on at least one adjustable variable using the quality parameter. The at least one adjustable variable may be the adjustable variable which is identified as having the highest correlation with the at least one quality parameter.

The determination of a calcination threshold prediction may be based on the identified indirectly adjustable variable and directly adjustable variable. The calcination threshold estimation may be obtained from a trained model. The trained model may be implemented using a gradient based tree regressor.

The method may comprise establishing a quality score based on the calcination threshold and the variation in quality. The quality score may be a linear combination of the calcination threshold and an value which represents the variation in quality.

Various steps may be repeated until successive quality scores differ by less than a threshold amount.

The method may further comprise outputting the calcination parameters corresponding to the step where the quality score differs from the previous quality score by less than the threshold amount.

A method in accordance with the second aspect provides a predictive model for predicting a calcination throughput using only adjustable variables and indirectly adjustable variables. This enables the calcination throughput to be optimised using historical calcination data.

Step vi) may comprise incrementing the adjustable variable and may repeat steps iii) to v) using the incremented value of the adjustable variable. This assists in converging on a parameters set which provides the optimal calcination throughput.

The method may further comprise configuring a calcination environment using the calcination parameters output in step vii). This means that the second aspect provides a way to predict an optimal calcination throughput and then the calcination environment can be configured in accordance with the calcination parameters.

Determining a variation in quality may comprise determining whether the quality drops beneath a threshold amount. The threshold amount may be specified by the calcination environment or by an operative. This provides the effect that the optimisation incorporates minimal boundaries on quality parameters such as, for example, moisture content.

The method may be initialised responsive to an operative of a calcination environment requesting an optimised configuration.

Computer programs and non-transitory storage mediums may be provided which implement the first and second aspects.

## DESCRIPTION

An embodiment of the invention is now described by way of example only and with reference to the following drawings in which:

Figure 1 schematically illustrates a prior art process of determining calcination throughput;

Figure 2 schematically illustrates a model of estimating calcination throughput in accordance with an embodiment of the invention;

Figure 2a illustrates the performance of the model on historical values of calcination throughput;

Figure 3 schematically illustrates a processing resource which may be used to implement an estimate of calcination throughput based on parameters received from a calcination environment;

Figure 4 schematically illustrates a flow of steps which may be used to estimate calcination throughput in accordance with an embodiment of the invention;

Figure 5 schematically illustrates a processing module used in the optimisation of a calcination environment; and

Figure 6 schematically illustrates a series of steps used in the optimisation of a calcination environment;

Figure 7 illustrates the performance of a model configured to predict an optimised configuration of a calcination environment;

Figure 8 illustrates the communication between the model configured to predict an optimised configuration and the calcination environment; and

Figure 9 illustrates a plurality of steps which shows how the calcination environment is configured using the optimised configuration.

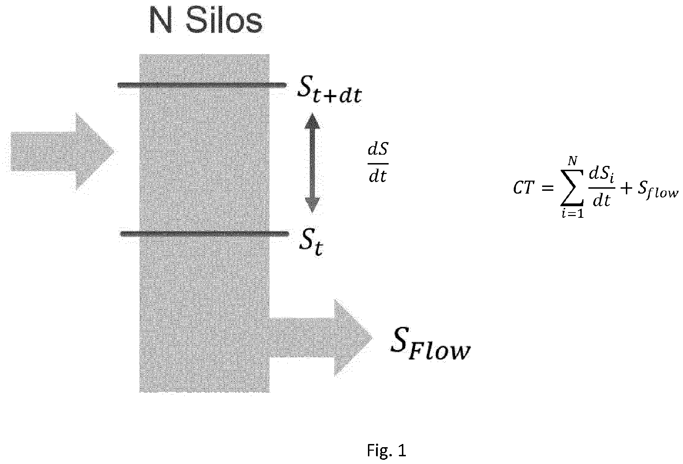

In prior art embodiments, estimating the amount of stucco which can be provided to a mixer during the production of plasterboard and other gypsum products is not directly evaluated using parameters taken in real-time at the time of production. Figure 1 schematically illustrates a silo for the storage of stucco which is to be used in the production of plasterboard.

In the prior art, the calcination throughput (CT) (in tonnes/hour) is estimated as the sum of the rate of change of the stucco level in each of the silos added to the current flow of the stucco going to the mixer ($S_{flow}$). More elegantly it is expressed as:

$$CT = \sum_{i=1}^{N} \frac{dS_i}{dt} + S_{flow}$$

The accuracy of this estimate is difficult to guarantee as the time derivative of the mass of stucco stored in the respective silos is difficult to calculate. It is generally approximated by comparing the level of stucco in each respective silo between two time stamps and taking the difference to be the time derivative (and averaging over the number of silos if N>1).

The difficulty comes from unreliable estimates of the amount of stucco stored in a silo and the need to denoise estimates by separating them over a sufficiently high amount of time, i.e. one hour, which causes information about the estimates between the one hour time instances to be lost.

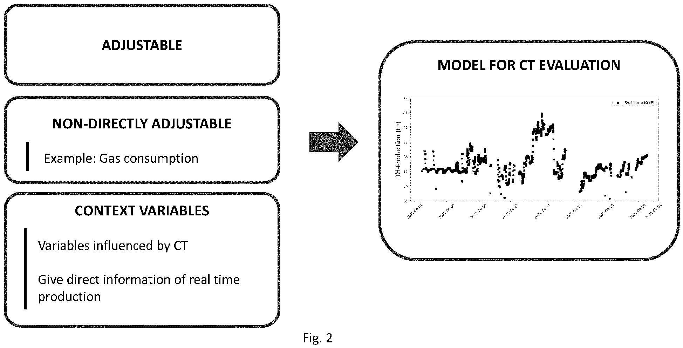

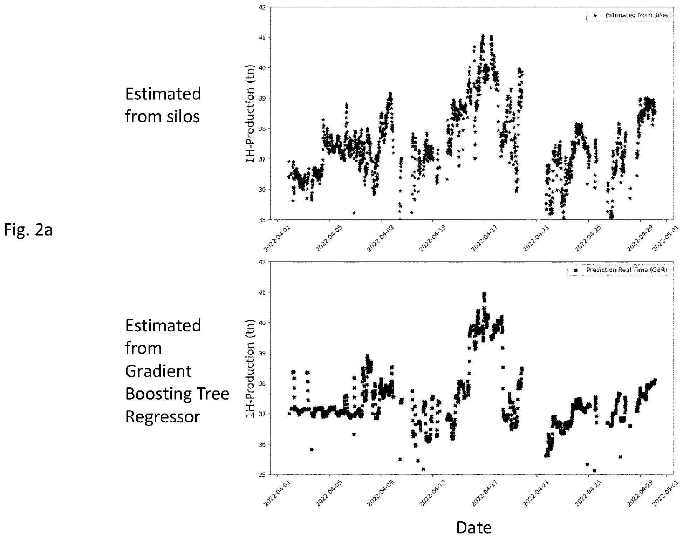

With respect to Figure 2, we schematically illustrate a model of calcination throughput estimation which we will be utilising in the described example. The model for CT evaluation, as described below, is trained on both directly and indirectly adjustable variables and context variables.

Trials analysing the correlation between parameters associated with the calcination environment and calcination throughput have been conducted to identify which parameters are most important in determining calcination throughput. The trials have been conducted on historical data taken from the calcination part of the production process used to produce plasterboard. Although the data related specifically to an environment used to make plasterboard, the calcination part, i.e. the part which is the focus of the data analysis, will be understood to be common to the production of other gypsum based products such as jointing compound or smoothing plaster.

The trials examined the parameters (which can be measured) over a 3 day period at a frequency of once every two minutes to establish the historical data which we could use to identify the correlation between the parameter and the calcination throughput.

The trials enabled the identification of the parameters which are significant when determining calcination throughput. Those variables were identified as being:

| Variable | Meaning | Unit |
|---|---|---|
| Gas Valve Percentage | The amount by which the gas inlet to the calcination environment is open | %, i.e. dimensionless |
| Gas consumption | The amount of gas which is being used by the calcination environment | Newton metres-s$^3$/hour |
| Output opening position | The amount by which the output of the calcination environment is open. | %, i.e. dimensionless |
| Combusion air temperature | The temperature of the air used for the gas combustion inside the calcination environment. | Degrees Centigrade |
| Recirculation air flow | The flow of air recirculating inside the calcination environment. | metre$^3$ /min |
| Main fan power | The amount of electricity used by the fan which is enabling the recirculation of air through the calcinator | Kilowatt |
| Rock feeding rate | A parameter which describes the amount of gypsum rock entering the calcination environment. It can be used to describe the speed of the system transporting rock to the calcinator. This is set relative to the maximum rate possible for the calcination equipment. | %, i.e. dimensionless |
| Calcination temperature | Temperature of the air inside the calcinatory. It provides an indication of how quickly the gypsum particles are heated to form stucco. | Degrees centigrade |
| Stucco cooler temperature | The temperature to which the stucco is cooled after calcination before going to the mixer - this may be inside a silo into which the stucco is placed before further mixing in the | Degrees centigrade |

(continued)

| Variable | Meaning | Unit |
|---|---|---|
| | production cycle. This is not a feature of every production environment. The silo may be smaller than the calcination environment and may impact calcination throughput. The cooling may be accelerated using a fan which is rotating and cooling the stucco | |
| Mill inlet pressure | Air pressure at inlet to calcinator | mBar |
| Mill outlet pressure | Air pressure at outlet to calcinator | mBar |
| Mill pressure | Air pressure inside calcinator. | mBar |

The variables can be categorised as directly adjustable, indirectly adjustable (i.e. those which will be adjusted as a direct result of the adjustment of the directly adjustable variables) and context variables.

The context variables are influenced by calcination throughput and provide information about real-time stucco production. The context variables may comprise mill outlet pressure, combustion air temperature, main fan power, the stucco cooler outlet temperature and the mill pressure.

The directly adjustable variables are mill inlet pressure, calcination temperature, gas valve percentage, the outlet opening position and the recirculation air flow. The indirectly adjustable variables, in this example, are the rock feeding rate and the gas consumption. The rock-feeding rate can be controlled by the calcination temperature. The gas consumption depends on the gas valve. The indirectly adjustable variables can depend on the specific production environments and the physical apparatus being configured. These parameters can be estimated using models which take adjustable parameters as input. Other parameters which have second order impacts may also be considered in the modelling, mainly the output opening position and the mill inlet pressure.

The directly adjustable variables and indirectly adjustable variables may change dependent on the specific apparatus being used at a plasterboard production facility. Some calcinators may, for instance, allow for a directly adjustable rock-feeding rate but only allow for a fixed mill inlet pressure.

The model illustrated in Figure 2 comprises a gradient boosting tree regressor. It is trained using by optimising an absolute error loss function where calcination throughput is used as a target variable and the variables set out in the table above are used as inputs. That is to say, production data associated with historical calcination production cycles is used as input to the training of the gradient boosting tree regressor and the labelled outputs are calcination throughput values. The labelled outputs, i.e. values of calcination throughput, are taken over a period of 6 months of measuring values of calcination throughput taken based on silo mass evaluation.

In this example, the gradient boosting tree regressor has 300 estimators, a learning rate of 0.5 and a max depth of 3. A dataset covering 6 months of calcination was utilised to provide the production data which is provided as input to the training of the gradient boosting tree regressor.

In this example, other parameters for the training of the gradient boosting tress regressor are:

| Parameter | Value |
|---|---|
| Subsample size, i.e. fraction of samples to be used for fitting the individual base learners | 1.0 |
| Criterion, i.e. the function to measure the quality of a split | Friedman Mean Squared Error |
| Minimum Number of Samples required to split an internal node | 2.0 |
| Minimum Number of Samples required to be at a leaf node | 1.0 |
| Minimum Weighted Fraction of the Sum Total of Weights of all the input samples required to be at a leaf node | 0 |
| Minimum Impurity Decrease, i.e. a node will be split if this split induces a decrease of the impurity greater than or equal to this value | 0 |
| Validation Fraction, i.e. proportion of training data to set aside as validation set for early stopping | 0.1 |
| Tolerance, i.e. tolerance for early stopping | 1e-4 |

These required parameters are guided by the requirements of the Python routine (sklearn.ensemble.Gradient-BoostingRegressor) and could change based on the approach to training a gradient boosting tree regressor.

That is to say, the model is trained to provide an estimate of calcination throughput based on input parameters which are associated with a calcination environment.

The model is tested by 4-fold cross validation on an entire dataset which describes calcination output over a period of 6 months. The dataset is distinct from the dataset used as the basis for the training of the model described above. Figure 2a shows how the model performed on the test dataset.

The upper figure corresponds to estimates using the prior art method set out above with reference to Figure 1. The lower figure corresponds to predictions using the gradient boosting tree regression model which is implemented according to the training approach set out above.

It can be seen that the curves are close to each other and the estimates provided by the trained model are close to what has been obtained in the test dataset. The mean absolute error between the curves is 0.56 tonnes.

In summary, the gradient boosting tree reqressor is trained based on historical values of calcination throughput to provide estimates of calcination throughput based on input parameters. The gradient boosting tree regressor is shown in Figure 2a to provide an effective estimate of calcination throughput based on calcination parameters.

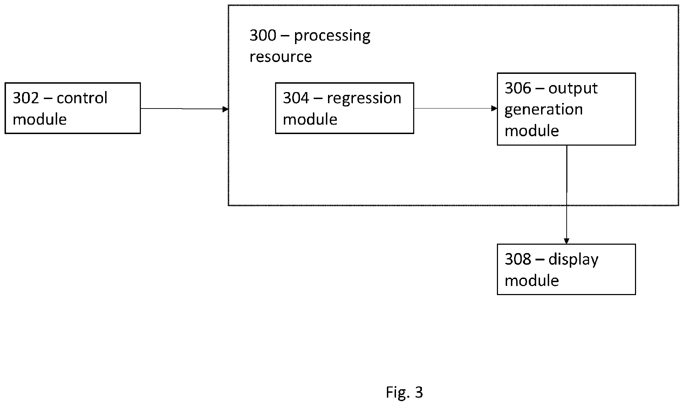

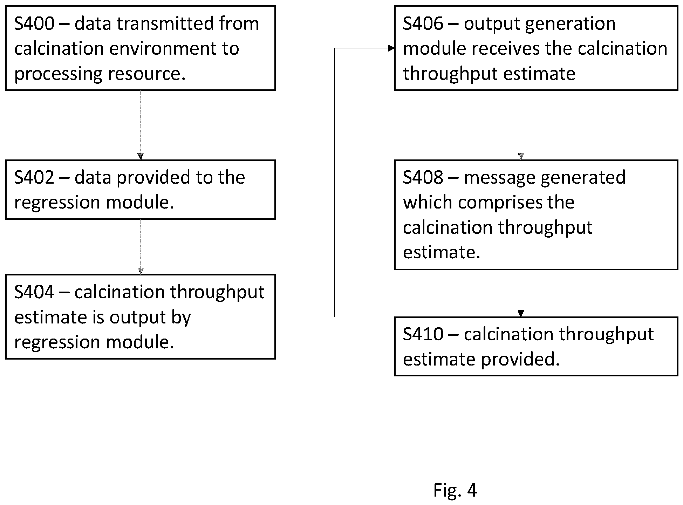

We will now describe, with reference to Figures 3 and 4, how a calcination throughput estimate can be provided in real-time during a production cycle.

In Figure 3, we schematically illustrate a processing resource 300 in relation to a calcination environment where calcination takes place during the production of plasterboard. An example of such a calcination environment may be a furnace or a calciner. The processing resource 300 interacts with a control module 302 of a calcination environment. The control module 302 is configured to control the adjustable variables of the calcination environment and is also configured to interact with sensors which take measurements from the calcination environment.

These measurements may be of gas valve percentage, gas consumption, output opening position, combustion air temperature, recirculation air flow, main fan power, rock feeding rate, calcination temperature, stucco cooler temperature, mill inlet pressure, mill outlet pressure and mill pressure. Other measurements of the calcination environment may also be taken and the control module may also be configured to interact with the sensors providing these other measurements.

The measurements can be taken from the calcination environment in real-time. The interval of the measurements can be specified by the configuration of the calcination environment or by an operative of the calcination environment. For example, it may be every second or minute or even every 2 minutes or another specified interval. The interval may be specified by an operative or a manufacturer of the calcination environment.

Processing resource 300 comprises a regression module 304 and an output generation module 306. Processing resource 300 is configured to receive data from the control module 302 via an input interface. The data comprises values determined from the calcination environment which may be values of gas valve percentage, gas consumption, output opening position, combustion air temperature, recirculation air flow, main fan power, rock feeding rate, calcination temperature, stucco cooler temperature, mill inlet pressure, mill outlet pressure and mill pressure. Other measurements of the calcination environment may also be taken and the processing resource 300 may be configured to receive data from these other measurements.

The regression module 304 is configured to implement the gradient boosted regressor described in relation to Figure 2 and Figure 2a. That is to say, it is configured to receive data from the calcination environment (i.e. the data provided by the control module 302) and provide an estimate of calcination throughput based on the received data using the gradient boosted regressor described in relation to Figure 2.

The output generation module 306 receives the estimate of calcination throughput from the regression module 304. It may be configured to store the estimate with a timestamp. The output generation module 306 then outputs the estimate to a display module 308.

The display module 308 is configured to provide a visual or audio output which conveys the calcination throughput estimate generated by the regression module 304.

The display module 308 may be coupled to the control module 302. The display module 308 may be co-located with the control module 302. The communication between the control module 302 and the processing resource 300 may be implemented using any suitable telecommunication protocol or media, i.e. the world-wide web or otherwise. The communication between the processing module 300 and the display module 308, i.e. when the calcination throughput estimate is provided by the output generation module 306 to the display module 308, can similarly be implemented using any suitable telecommunication protocol or media such as, for example, the world-wide web.

In other words, the processing resource 300 need not be co-located with any of the components of the calcination environment. The processing resource 300 may be cloud implemented. The components of the processing resource 300 may be distributed over various locations.

In a step S400, shown in Figure 4, data is transmitted from the calcination environment to the processing resource 300 via the control module 302. The transmittal of data between the calcination environment and the processing resource may take place at a frequency which is specified by an operative of the calcination environment or at a frequency which is specified by the calcination environment. The frequency may be specified in terms of seconds or minutes to enable the estimate of calcination throughput to be provided in real-time. That is to say, the data may be transmitted from the control module 302 every minute, every 2 minutes or at any other time interval which could be specified by the manufacturer of the calcination environment, an calcination environment operative or a production line operative (in the plasterboard production environment).

The data transmitted may comprise one or more parameters which are associated with conditions inside the calcination environment. The data may comprise any one or more measurements of variables including the mill inlet pressure, the calcination temperature, gas consumption, the gas valve percentage, the output opening position, the mill outlet pressure, the combustion air temperature, the rock feeding rate, the recirculation air flow, the main fan power, the stucco cooler outlet temperature and the mill pressure. In some calcination environments, it may be that some parameters are being monitored and controlled and the remaining parameters are taken to be stable, i.e. if the control module 302 determines that they have not varied appreciably (i.e. not changed outside of a specific tolerance) in the previous 10 minutes (or over another specified time interval) and only those monitored and controlled parameters are sent by the control module 302 to the processing resource 300 as the other parameters (i.e. those which are stable) are already at the processing resource 300.

The data may be transmitted responsive to an operator providing input requesting a real-time calcination throughput estimate. Alternatively or additionally, the request may be transmitted at a frequency specified by the control module 302 or by the manufacturer of the calcination environment. The request may be transmitted in real-time (i.e. second-by-second or more frequent).

In one example, the variables may be separated as directly adjustable variables, indirectly adjustable variables and context variables. The directly adjustable variables may include the mill inlet pressure, the calcination temperature, gas valve percentage, output opening position and recirculation air flow. The indirectly adjustable variables are any value than can be directly influenced by one of the directly adjustable variables. Examples of indirectly adjustable variables include the gas consumption which is directly influenced by the gas valve percentage. The context variables are those variables which are influenced by calcination throughput and give direct indicators of the real-time production.

In a step S402, the data received from the control module 302 is provided to the regression module 304.

In a step S404, the regression module 304 outputs a calcination throughput estimate for the provided data using the trained model implemented therein.

The regression module 304 may alternatively implement a different model (to the gradient boosting tree regression model described above). The model described in relation to Figure 2 utilises a gradient boosting tree regressor as described above. However, other approaches may also be deployed. A standard ANN topology may also deployed which takes the respective calcination parameters as input and provides the calcination throughput estimate as an output. Such an ANN may be trained on historical production data which provides the calcination parameters as an input with the output as the respective calcination throughput estimates taken during the historical production cycle to which the production data pertains.

In a step S406, the output generation module 306 receives the calcination throughput estimate from the regression module 304. The output generation module 306 may be configured to determine from the calcination throughput estimate whether it falls within acceptable values of calcination throughput. A calcination throughput which is too low may indicate a problem in the calcination environment which requires attention. This may lead to the generation of an alarm in the production environment surrounding the calcination environment. A calcination throughput which is too high may indicate that the production environment may need adjustment to avoid wastage if the plasterboard production cannot work fast enough. An alarm may also be generated if the calcination throughput estimate is higher than a specified maximum. A notification may be transmitted to a computing device assigned to a designated operative to confirm a calcination throughput estimate which is too low or too high. A notification that the calcination throughput is too high or too low may also generate a failsafe message which is transmitted back to the control module 302 with a request that the calcination environment is powered-off.

In a step S408, the output generation module 306 generates a message which is to be transmitted to the display nodule 308. The message comprises the calcination throughput estimate.

In a step S410, the calcination throughput estimate may be displayed visually as a visual notification by the display module 308 or provided as an audio notification via the display module 308. This notification is provided if preferences of a user or operative indicate they would like it to be provided to them. The notification may be provided to a computing device in communication with the processing resource 300.

Optionally or additionally, the calcination throughput estimate may then be provided to an operative or user who can use the display module 308 or can perceive the output of the display module 308.

Steps S400 to S408 may be repeated every time data is transmitted from the calcination environment to the processing resource via the control module 302. Steps S400 to S408 may be repeated at an interval specified by a user or operative. Step S410 may be repeated at intervals specified by preferences of a user or operative. For example, the preferences may specify that the calcination throughput estimate is displayed or notified to the user or operative at a specified frequency. The calcination throughput estimate may be provided on request to the user or operative via a suitable user interface. The user interface may be accessed using a suitable application using a computing device in communication with the processing resource 300. This enables the calcination throughput estimates to be obtained in real-time and then provided to operatives and users who are operating or working in association with the calcination environment. This enables the calcination throughput to be estimated accurately in real-time.

The calcination throughput estimate may be stored every time it is produced in step S404. The estimate may be stored in local storage or remote storage.

That is to say, as described in steps S400 to S410, we have trained a model which can receive input parameters from a calcination environment and provide a calcination throughput estimate in real-time. In other words, the processing resource 300 provides a real-time estimate of calcination throughput based on process parameters which are taken from the calcination environment in real-time without the unreliability of the estimates described in the prior art. The trained model is trained on data taken from a calcination environment over a long time period, e.g. 6 months, which is long enough to integrate many different production scenarios.

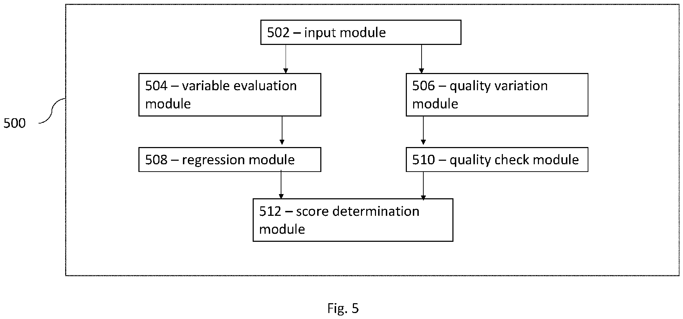

We now describe, with reference to Figure 5, a processing resource 500 which can generate a configuration for a calcination environment which can optimise the calcination throughput.

The processing resource 500 comprises an input module 502, a variable evaluation module 504, a quality variation module 506, a regression module 508, a quality check module 510 and a score determination module 512. The respective modules are configured to communicate with each other using any suitable telecommunications medium or protocol. The modules may be located in the same location or my be located remotely relative to each other. The processing resource 500 may be implemented using software or hardware resources. The processing resource 500 may be implemented through the cloud.

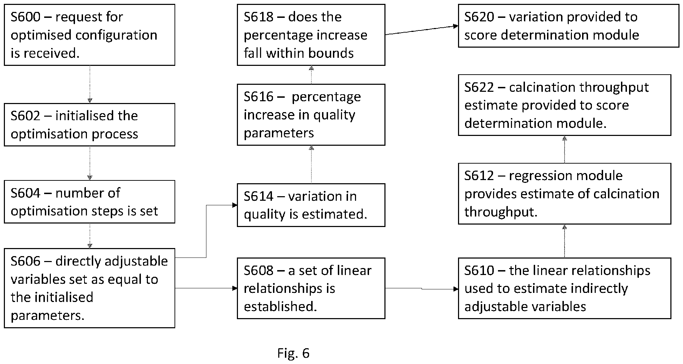

The interaction between these modules will be described in reference to Figure 6 where it is described how the processing resource 500 generates a configuration which can optimise calcination throughput from a calcination environment.

In a step S600, a request is received at the input module 502 for an optimum configuration of a calcination environment from control unit 302. The request may be as a result of user input from an operative of the calcination environment. The request may be generated automatically by the control unit 302, i.e. without user input. The requests may be generated at a specific frequency which is specified by a user or by a setting in the control unit. That is to say, the optimum configuration may be determined at such a frequency and provided as an update as measurements of respective parameters are received from the calcination environment or user input, i.e. the user may determine that they would like , for example, output from the calcination environment which is of a specific quality and the optimisation may be focused on quality parameters such as combined water and AIII.

In a step S602, the input module 502 initialises the optimisation process. The initialisation comprises retrieving ranges on the adjustable variables (both directly and indirectly adjustable variables) which define the acceptable ranges for those variables , an initialised set of parameters corresponding to the adjustable variables (e.g. initial values for the adjustable variables for the calcination environment which is subject of the optimisation) and maximum deviations from quality parameters which are acceptable for the production process. The initialised set of parameters comprise measurements of those adjustable variables which have the highest correlation with quality parameters. Example quality parameters are combined water (the quantity of water associated with the produced stucco) and AIII (the concentration of over-calcined gypsum particles in the stucco). The initialised set of adjustable parameters may comprise, for example, one or more of the mill inlet pressure, the calcination temperature, the gas valve percentage, the output opening position and the recirculation air flow.

In the example based on combined water and AIII, analysis of historical calcination data has determined that the output opening position and the calcination temperature have the highest correlation with combined water and AIII individually. In other examples, other variables may be identified by analysis of historical calcination data as having the highest correlation with combined water and AIII. Additionally, where other quality parameters are identified, i.e. other than combined water and AIII, other variables may be identified by analysis of historical calcination data as having the highest correlation with the identified quality parameters. Therefore, the initialised set of adjustable parameters will comprise at least initial values on output opening position and the calcination temperature. In other examples the initialised set of adjustable parameters will comprise initial values on other variables e.g. mill inlet pressure, gas valve percentage and recirculation air flow.

In a step S604, a number of iterations for the optimisation process is set. This is the number within which a convergence on a set of parameters is expected. The number of iterations could be described as the maximum number of optimisation steps. For example, this number could be set to 100 to provide sufficient scope for convergence to take place. This number could be adjusted by an operative. For example, if the operative had a set of initialised parameters which they believed were close to optimal, then the number of optimisation steps could be set to less than 100. However, if the purpose was more experimental, e.g. product development or development of the calcination environment, the number of optimisation steps could be set to more than 100.

In a step S606, the first set of directly adjustable variables (for the optimisation analysis) is set at the initialised set of parameters at step S602. The number of the optimisation step is set to 1. The first set of directly adjustable variables is then provided to the variable evaluation module 504 and the quality variation module 504.

In a step S608, a set of linear relationships between directly adjustable variable and indirectly adjustable variables is obtained using, for example, Ridge Regression. This is implemented using the variable evaluation module 504. This makes the assumption that there is a linear relationship between a respective directly adjustable and indirectly adjustable variable. The parameters of the linear relationship between the directly adjustable variable and the indirectly adjustable variable are then estimated by the Ridge Regression. For example, a linear relationship between gas consumption (as the indirectly adjustable variable) and the gas valve percentage (as the directly adjustable variable) is estimated. In another example, a linear relationship between the rock feeding rate (as the indirectly adjustable variable) and the calcination temperature (as the directly adjustable variable) is estimated.

In other examples, it may be determined whether there is a linear relationship between a directly adjustable variable and an indirectly adjustable variable by determining the correlation coefficient between the two variables based on the historical calcination data. The parameters of the linear relationship may then be determined using Ridge Regression.

Step S608 may be executed on historical data (e.g. historical calcination data) for a given production period, e.g. the previous 6 months. That is to say, Ridge Regression may be applied to the historical calcination data to establish linear relationships between indirectly and directly adjustable variables.

Such data may be separated into a fitting set and a test data set. The parameters may be estimated using the fitting set and the resulting model may then be tested and refined using the test data set.

Other methods of estimating the parameters of a linear relationship between the direct and indirectly adjustable variables may also be deployed, e.g. linear regression.

In a step S610, the linear relationships between the directly adjustable variables and the indirectly adjustable variables are then used to estimate the indirectly adjustable variable from the value of the directly adjustable variable provided in step S606 in the initialised set of parameters.

The directly adjustable variables and the newly estimated indirectly adjustable variables are then provided to the regression module 508 with a request for an estimate of calcination throughput.

The regression module 508, similar to the regression module 304, deploys a gradient boosting tree regression model which is trained on historical data from the calcination environment (e.g. historical calcination data) to provide a calcination throughput estimate based on the provided parameters. It is distinct from the regression module 304 in that it is not trained on context variables as those context variables are influenced by the calcination throughput and would therefore bias the optimisation process.

The gradient boosting regression model deployed by regression module 508 is trained using 300 estimators, a learning rate of 0.5 and maximum depth of 2. The other parameters are the same as those used in the training of the gradient boosting regression model deployed by regression module 304.

In a step S612, the regression module 508 provides a calcination throughput estimate based on the directly adjustable variables and the estimated indirectly adjustable variables which are estimated by the variable evaluation module in step S610.

Simultaneously or contemporaneously to steps S604 to S612, in a step S614, the quality variation module 506 is used to determine whether the directly adjustable variables lead to a variation in quality. Step S614 may also be carried out after step S612 is completed.

In the example based on combined water and AIII, analysis of historical calcination data has determined that the output opening position and the calcination temperature have the highest correlation with combined water and AIII individually, i.e. the quality parameters. That is to say, it is determined there are linear relationships between output opening position and the calcination temperature.

In step S616, the quality variation module 504 determines the percentage increase in combined water and AIII provided by the output opening position and the calcination temperature. The percentage increase is determined relative to the previous optimisation step. That is to say, the percentage increase in quality of the produced stucco is calculated based on increases or decreases in the adjustable variables which are determined to correlate most strongly with the quality parameters. In other examples, i.e. examples which are not based on combined water and AIII, other adjustable variables may be determined to correlate most strongly with the quality parameters and used to estimate linear relationships which connect adjustable parameters with quality parameters.

The percentage increase is determined based on historical calcination data. It may be the same historical calcination data used in step S614. Alternatively or additionally, it may be a different set of historical calcination data from the same production period. Where corresponding values of the directly adjustable variables cannot be found, an estimate may be extrapolated from the determined linear relationships determined in step S614. In this example, such a linear relationship may connect output opening position and the calcination temperature (as determined in step S614) and percentage increases in quality may be determined based on extrapolation from that linear relationship.

In step S618, the quality check module 510 determines whether the percentage increase in quality are within allowable variations in quality. That is, in an example where combined water and AIII are the quality parameters of most importance, this step determines whether resultant levels of combined water and AIII are within allowable variations in order to maintain a quality level.

If the change in combined water and AIII falls outside of allowable variations, the processing module 500 may be configured to issue a rejection message to the terminal which issued the request in step S600.

In subsequent iterations, the change in the quality parameters (in this example, combined water and AIII) may be compared with the previous iteration and the set of parameters corresponding to the adjustable variables provided at the start of the current iteration may be rejected if they provide lower quality than the previous iteration.

If the change in combined water and AIII is within the range of allowable variations, the quality check module 510 provides the respective variation in combined water and AIII to the score determination module 512. This is step S620.

The variation may be estimated based on base line values of the quality parameters (e.g. base line values of combined water and AIII) associated with the initial running conditions of the calcination environment in question. This means that calcination environments are being explored are based on predictions which incorporate the calcination environment and the base line quality parameters set by that calcination environment. This means that, for instance, values of output opening position or calcination temperature which lead to combined water which increases too much from the base line for the specific calcination environment being optimised can be rejected as a possibility.

In a step S622, the regression module 508 provides the estimate of calcination throughput (CT) to the score determination module 512.

The score determination module 512 then calculates a score based on a sum of -CT + Quality Variation.

- CT should be understood as the negative of the calcination throughput estimate provided by step S622.

The quality variation may be described as the average percentage increase (or decrease) in combined water and AIII.

In the next iteration, the output opening position and the calcination temperature are incremented. These parameters are chosen in this example because, as noted above, it is found that these parameters correlated most strongly to the quality parameters of combined water and AIII.

In the case of the output opening position, it is increased by, for example, a centremetre. In the case of the calcination temperature, it is increased by, for example, 0.5 degrees centigrade. The steps S606 to S622 are then run again.

Convergence on an optimised set of parameters is determined to be found when successive scores determined by the score determination module 512 differ by a sufficiently small number (e.g. determined by an operative or by modelling using an objective function which is focused on maximisation of quality. A typical value of such a number of 0.01

When convergence is achieved, the corresponding set of parameters is output as the optimised configuration of the calcination environment. The optimised configuration provides a calcination environment which maintains quality requirements and calcination throughput.

That is to say, steps S606 to S622 are then repeated up until the processing resource 500 reaches the number of iterations specified in step S604. If the score determines by the score determination module 512 is lower than a specified convergence threshold before the number of iterations is reached then the optimisation stops.

When convergence is reached the values of the adjustable variables (in this example output opening position and calcination temperature) are output as the optimum adjustable parameter set. We will describe below how these values can be used in the calcination environment to provide an optimised calcination environment.

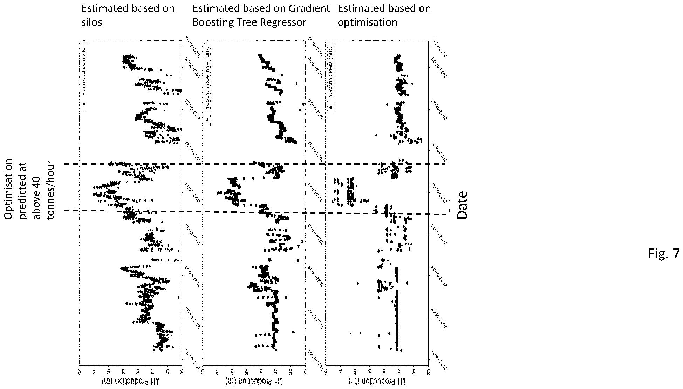

The performance of the processing resource 500 is similarly tested on test data using 4-fold cross validation (for the regression module 504). The test data comprises calcination data from production over a 6 month period. Figure 7 shows the predictions provided by the processing module 500 over an example testing period of 1.5 months. The production of stucco is predicted by the prior art estimate (top figure), the gradient boosting tree regressor (middle figure) and processing module 500 (bottom figure).

The mean absolute error between the optimum predicted by the processing module 500 and the silo-based prediction is 0.7 tonnes/hour. It can be seen that the processing module 500 detects the production peak where calcination throughput was predicted to be above 40 tonnes/hour.

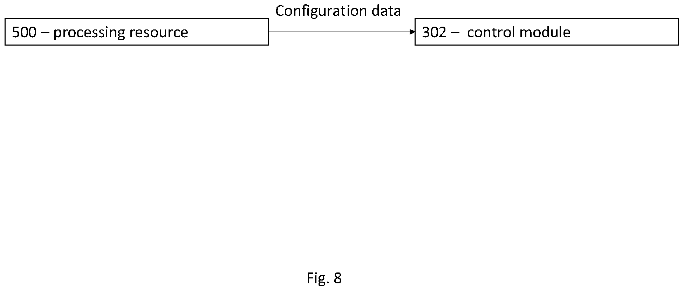

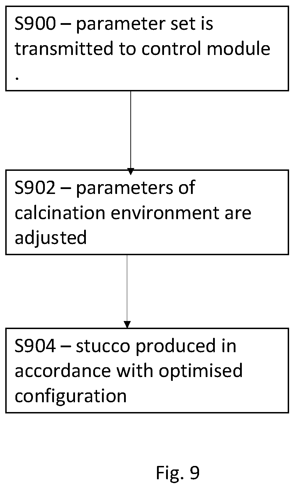

We will now describe, with reference to Figures 8 and 9, how the optimised configuration which is generated by the processing resource 500 can be used to provide an optimised calcination environment. In a step S900, upon output by the processing resource 500 of the optimum adjustable parameter set, the optimum adjustable parameter set is transmitted as configuration data to the control module 302 described above. As set out above, the control module 302 is configured to control the adjustable variables of the calcination environment and is also configured to interact with sensors which take measurements from the calcination environment.

The transmission of the configuration data to the control module 302 may be automatic (i.e. without the need for human input or intervention) or responsive to a user input from a user who is prompted via a user interface to proceed with calcination based on the configuration which is predicted to be optimal by the processing resource 500.

The control module 302 uses the parameters in the configuration file to adjust the respective adjustable variables of the calcination environment being controlled by the control module 302. That is to say, the control module 302 may receive values for those parameters which are determined to correlate most strongly with quality parameters and use those values to configure the calcination environment for optimal performance. In this example, this means the output opening position and the calcination temperature are adjusted to those predicted to be optimal by the processing resource 500 in steps S600 to S622. This is step S902. The control module 302 may also similarly adjust the other parameters such as, for example, the mill inlet pressure, the gas valve percentage and the recirculation air flow.

Stucco can then be produced in accordance with the optimised configuration which is predicted by the processing resource 500 in steps S600 to S622. This is step S904.

The stucco can be used in the production of plasterboard products such as plasterboard and powder products.

The processing resource 500 may be used to realise distinct production scenarios where an optimal configuration can be predicted using differing constraints on quality parameters.

In a "secure" case, constraints were imposed on the combined water of 5.4% and the AIII of 8.8% and it was used to realise a calcination throughput of 38-39 tonnes per hour based on a configuration predicted by processing resource which set the mill inlet pressure at -8 mbar, the calcination temperature at 154.6 degrees Celsius, the gas valve percentage at 75% , the output opening position at 16 cm and the recirculation air flow at 2350 cubic metres/minute.

The output opening position and the calcination temperature were then increased(to respectively 17 cm and 155.4 degress Celsius) and the constraints on the combined water and AIII were adjusted (to 5.4% and 7.8%-8.7% respectively) and this realised a calcination throughput of 39-40 tonnes per hour.

That is to say, optimisation using the processing resource 500 and by adjusting the minimum bounds on quality parameters can be used to predict increases or decreases in calcination throughput. We have described the estimation of calcination throughput and the optimisation of calcination throughput using a data-driven approach. We have shown that real-time estimation of calcination throughput and real-time optimisation of calcination throughput can be implemented using a reliable model which enables estimation of calcination throughput and optimisation of calcination throughput to be realised.

It should be noted that the above-mentioned aspects and embodiments of the invention illustrate rather than limit the disclosure, and that those skilled in the art will be capable of designing many alternative embodiments of the invention without departing from the scope of the disclosure as defined by the appended claims. In the claims, any reference signs placed in parentheses shall not be construed as limiting the claims. The word "comprising" and "comprises", and the like, does not exclude the presence of elements or steps other than those listed in any claim or the specification as a whole. In the present specification, "comprises" means "includes or consists of" and "comprising" means "including or consisting of". The singular reference of an element does not exclude the plural reference of such elements and vice-versa. The disclosure may be implemented by means of hardware comprising several distinct elements, and by means of a suitably programmed computer. In a device claim enumerating several means, several of these means may be embodied by one and the same item of hardware. The mere fact that certain measures are recited in mutually different dependent claims does not indicate that a combination of these measures cannot be used to advantage.

**Claims**

1. A computer-implemented method of estimating calcination throughput from a calcination environment, the method implemented by a processing resource, the method comprising:

   initialising a trained model, the model trained to predict a calcination throughput from a input associated with a calcination environment, wherein the model is trained using historical calcination data;
   receiving at least one calcination parameter associated with the calcination environment;
   applying the trained model to the at least one calcination parameter; and
   obtaining, from the trained model, a calcination throughput estimate for the calcination environment by using the at least one calcination parameter as an input to the trained model.

2. The method of Claim 1, wherein the trained model comprises a regression based estimator.

3. The method of Claim 2, wherein the regression based estimator comprises a gradient boosting tree regressor.

4. The method of any preceding claim, wherein the training of the trained model comprises supervised learning where production parameters associated with calcination environments and calcination throughputs are used as respective labelled input-output pairs.

5. The method of any preceding claim, wherein the at least one calcination parameter comprises at least one of mill inlet pressure, calcination temperature, gas consumption, output aperture position, mill outlet pressure, combustion air temperature, rock feeding rate, recirculation air flow, main fan power, stucco cooler outlet temperature and mill pressure.

6. The method of any preceding claim, wherein the at least one calcination parameter is obtained autonomously from the calcination environment.

7. The method of any preceding claim, wherein the at least one calcination parameter is obtained from the calcination environment responsive to human input.

8. The method of any one of Claims 6 or 7, wherein the calcination throughput estimate is provided to a display.

9. A computer-implemented method of generating a configuration for a calcination environment, the method implemented by a processing resource, the method comprising:

obtain historical calcination data, wherein the historical calcination data comprises measurements related to a plurality of directly adjustable and indirectly adjustable variables;
converge on a set of optimised calcination parameters by:

i) identifying relationships between at least one directly adjustable variable and an indirectly adjustable variable based on the historical calcination data;
ii) identifying a correlation between at least one directly adjustable variable and a quality parameter
iii) determining a variation in quality in the plasterboard based on at least one adjustable variable using the quality parameter;
iv) determining a calcination threshold prediction based on the identified indirectly adjustable variable and directly adjustable variable;
v) establishing a quality score based on the calcination threshold and the variation in quality;
vi) repeating steps iii) to v) until successive quality scores differ by less than a threshold amount;
vii) outputting the calcination parameters corresponding to the step where the quality score differs from the previous quality score by less than the threshold amount.

10. A method according to Claim 9, wherein step vi) comprises incrementing the adjustable variable and repeating steps iii) to v) using the incremented value of the adjustable variable.

11. A method according to Claim 9 or 10, wherein the method further comprises configuring a calcination environment using the calcination parameters output in step vii).

12. A method according to any one of Claims 9 to 11 wherein determining a variation in quality comprises determining whether the quality drops beneath a threshold amount.

13. A method according to any one of Claims 9 to 12 wherein the method is initialised responsive to an operative of a calcination environment requesting an optimised configuration.

14. A computer-program product which, when executed on a processing medium, configures the processing medium to implement the steps of any one of Claims 1 to 9.

15. A non-transitory storage medium configured to store instructions which, when executed by suitably configured hardware, provides instructions to a processing medium to implement the steps of any one of Claims 1 to 9.

N Silos

$$CT = \sum_{i=1}^{N} \frac{dS_i}{dt} + S_{flow}$$

$S_{t+dt}$

$\frac{dS}{dt}$

$S_t$

$S_{Flow}$

Fig. 1

ADJUSTABLE

NON-DIRECTLY ADJUSTABLE

Example: Gas consumption

CONTEXT VARIABLES

Variables influenced by CT

Give direct information of real time production

MODEL FOR CT EVALUATION

Fig. 2

Fig. 2a

Estimated
from silos

Estimated
from
Gradient
Boosting Tree
Regressor

Date

300 – processing
resource

302 – control
module

304 – regression
module

306 – output
generation
module

308 – display
module

Fig. 3

S400 – data transmitted from calcination environment to processing resource.

S402 – data provided to the regression module.

S404 – calcination throughput estimate is output by regression module.

S406 – output generation module receives the calcination throughput estimate

S408 – message generated which comprises the calcination throughput estimate.

S410 – calcination throughput estimate provided.

Fig. 4

Fig. 5

S600 – request for optimised configuration is received.

S602 – initialised the optimisation process

S604 – number of optimisation steps is set

S606 – directly adjustable variables set as equal to the initialised parameters.

S618 – does the percentage increase fall within bounds

S616 – percentage increase in quality parameters

S614 – variation in quality is estimated.

S608 – a set of linear relationships is established.

S620 – variation provided to score determination module

S622 – calcination throughput estimate provided to score determination module.

S612 – regression module provides estimate of calcination throughput.

S610 – the linear relationships used to estimate indirectly adjustable variables

Fig. 6

Fig. 7

Configuration data

| 500 – processing resource | | 302 – control module |

Fig. 8

S900 – parameter set is transmitted to control module .

S902 – parameters of calcination environment are adjusted

S904 – stucco produced in accordance with optimised configuration

Fig. 9

Europäisches
Patentamt

European
Patent Office

Office européen
des brevets

## EUROPEAN SEARCH REPORT

**Application Number**

EP 24 30 6487

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHENG J. ET AL: "Modeling and Optimization of the Cement Calcination Process for Reducing NOx Emission Using an Improved Just-In-Time Gaussian Mixture Regression", INDUSTRIAL & ENGINEERING CHEMISTRY RESEARCH, vol. 59, no. 11, 14 February 2020 (2020-02-14), pages 4987-4999, XP093250701, ISSN: 0888-5885, DOI: 10.1021/acs.iecr.9b05207 * the whole document * ----- | 1-15 | INV. G16C20/10 ADD. G16C20/70 G16C60/00 |
| A | KAHAWALAGE A. C. ET AL: "Modelling a Cement Precalciner by Machine Learning Methods", PROCEEDINGS OF THE FIRST SIMS EUROSIM CONFERENCE ON MODELLING AND SIMULATION, SIMS EUROSIM 2021, AND 62ND INTERNATIONAL CONFERENCE OF SCANDINAVIAN SIMULATION SOCIETY, SIMS 2021, SEPTEMBER 21-23, VIRTUAL CONFERENCE, FINLAND, vol. 185, 31 March 2022 (2022-03-31), pages 99-106, XP093250834, DOI: 10.3384/ecp2118599 ISBN: 978-91-7-929219-5 Retrieved from the Internet: URL:https://ecp.ep.liu.se/index.php/sims/article/download/333/291> * the whole document * ----- -/-- | 1-15 | TECHNICAL FIELDS SEARCHED (IPC) G16C G06N E04B E04C |

The present search report has been drawn up for all claims

| Place of search | Date of completion of the search | Examiner |
|---|---|---|
| The Hague | 20 February 2025 | Godzina, Przemyslaw |

EPO FORM 1503 03.82 (P04C01)

| | Europäisches Patentamt |
| European Patent Office |
| Office européen des brevets |

# EUROPEAN SEARCH REPORT

**Application Number**

EP 24 30 6487

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JACOBS M. ET AL: "Real-time gypsum quality estimation in an industrial calciner: A neural network-based approach", JOURNAL OF THE SOUTHERN AFRICAN INSTITUTE OF MINING AND METALLURGY, vol. 123, no. 10, 1 October 2023 (2023-10-01), pages 483-490, XP093249876, Johannesburg, South Africa ISSN: 2225-6253, DOI: 10.17159/2411-9717/2480/2023 * the whole document * ----- | 1-15 | |
| A | CN 115 392 007 A (CFHI GROUP DALIAN ENG TECH CO LTD; CHINA FIRST HEAVY INDUSTRIES) 25 November 2022 (2022-11-25) * the whole document * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 20 February 2025 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 30 6487

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2025

| Patent document cited in search report | Publication date | Patent family member(s) | Publication date |
|---|---|---|---|
| CN 115392007 A | 25-11-2022 | NONE | |

EPO FORM P0459

For more details about this annex : see Official Journal of the European Patent Office, No. 12/82